# FASCICULE DE BREVET EUROPEEN

(11) **EP 1 643 916 B1**
(45) Date de publication et mention de la délivrance du brevet: **17.03.2010**
(21) Numéro de dépôt: 04767685.3
(22) Date de dépôt: 15.07.2004
(51) Int. Cl.: A61B 17/15

(54) **DISPOSITIF D'AIDE POUR L'IMPLANTATION DE PROTHESES TOTALES DU GENOU**
VORRICHTUNG ZUR UNTERSTÜTZUNG BEI DER IMPLANTATION EINER KNIETOTALPROTHESE
DEVICE FOR ASSISTING IN TOTAL KNEE PROSTHESIS IMPLANTATION

(30) Priorité: 16.07.2003 FR 0308698
(43) Date de publication de la demande: 12.04.2006
(73) Titulaire: Depuy (Ireland) Limited, Ringaskiddy, County Cork (IE)
(72) Inventeur: SCHIFRINE, Patrick, F-74290 Veyrier du Lac (FR); FORNASIERI, Christophe, F-38240 Meylan (FR); VIE, Pascal, F-76130 Mont Saint Aignan (FR); LE SAOUT, Jacques, F-29870 Landeda (FR); MUSSET, Thierry, F-56260 Larmor Plage (FR); LAURENT, Patrick, F-47270 Puymirol (FR); TRILLAUD, Jean-Marie, F-74550 Perrignier (FR); DUCASSE, Philippe, F-64100 Bayonne (FR); TARQUINI, Cyril, F-26740 Sauzet (FR); GIROU, Yves, F-85000 La Roche sur Yon (FR); BOSREDON, Jean-Léon, F-33200 Bordeaux (FR); LOOTVOET, Louis, B-5000 Namur (BE); MULLIER, Jean, B-1360 Perwez (BE); HIMMER, Olivier, B-5004 Bouge (BE); FORTHOMME, Jean-Paul, B-7030 St Symphorien (BE); BREYSSE, Michael, F-69790 Saint-Pierre de Chandieu (FR)
(74) Mandataire: Lagrange, Jacques Etienne M.M.
(86) Numéro de dépôt international: PCT/FR2004/001861
(87) Numéro de publication internationale: WO 2005/006993

(56) Documents cités:
- WO-A-98/25526
- FR-A- 2 776 176
- FR-A- 2 806 901
- US-A- 5 486 178

## Description

La présente invention a trait à un dispositif d'aide pour prothèse totale du genou, destiné à assurer la mise sous tension de l'articulation et à faciliter les opérations de coupe et d'équilibre pour le praticien pendant l'intervention.

L'invention a également pour objet les- procédés chirurgicaux de pose de prothèse totale du genou mettant en oeuvre ce dispositif.

On connaît déjà un certain nombre de dispositifs auxiliaires destinés à guider le chirurgien pendant la pose d'une prothèse totale du genou, et, notamment, à l'aider à déterminer les plans de coupe. On rappellera, à ce sujet, que la pose d'une prothèse nécessite, outre la coupe tibiale au niveau du plateau pour recevoir le composant tibial de la prothèse, une coupe distale du fémur qui appuie sur le composant fémoral de la prothèse, ainsi qu'une coupe postérieure du fémur au niveau des condyles et, le cas échéant, d'autres coupes dont une coupe antérieure.

Ainsi des dispositifs auxiliaires sont décrits dans EP-A-0327249 et FR 2 806 901. Le document EP 0 979 636 décrit un dispositif pour tendre les ligaments du fémur et pour déterminer la taille d'une prothèse.

Un dispositif perfectionné est décrit dans le document WO98/25526, ce dispositif comprenant une pièce ancillaire présentant une plaque tibiale suivie d'une tige centro-médullaire destinée à être introduite dans le canal médullaire fémoral, cette plaque étant susceptible de recevoir des moyens de calage, d'épaisseurs variables destinés à prendre appui sur le plateau tibial après la coupe tibiale pour, d'une part, permettre de mesurer et d'équilibrer la laxité de l'articulation du genou en extension, d'autre part, déterminer l'emplacement de la coupe fémorale postérieure, en flexion, pour assurer un équilibre articulaire sous une tension ligamentaire convenable dans toutes les positions du genou allant de l'extension à la flexion complète.

Les moyens de calage, d'épaisseur variable, sont tels que l'épaisseur totale de la plaque et de la cale soit égale à la distance entre le plan de coupe proximal tibial et l'extrémité distale du fémur, la plus proche après obtention de l'équilibre ligamentaire par distraction de l'articulation entre la plaque et l'extrémité distale du fémur.

La présente invention se propose de fournir un dispositif de mise sous tension pour prothèse totale du genou, qui permette, d'une façon simple, de mettre le genou en tension ligamentaire dans les positions naturelles, de déterminer éventuellement la position du plan de coupe fémoral distal et de déterminer la position du plan de coupe fémoral postérieur, ainsi que du plan de coupe fémoral antérieur, et ceci d'une façon très simple et pratique pour le chirurgien.

Un autre objectif de l'invention est de permettre, en même temps, d'estimer la taille de la prothèse fémorale qui doit être posée de façon optimale.

Un autre objectif encore de l'invention est de fournir un tel dispositif qui permette une mise en place optimale des guides de coupe à l'extrémité distale du fémur, tout en étant susceptible d'assurer les fonctions précitées au cas où le chirurgien préfère commencer par procéder d'emblée à une coupe fémorale distale.

L'objectif global du dispositif est de permettre de réaliser les coupes en fonction de la prothèse choisie, pour assurer un équilibrage ligamentaire de très bonne qualité sur l'ensemble de la course de flexion du genou.

L'invention a pour objet un dispositif pour pose d'une prothèse totale du genou comportant :
une pièce de mise sous tension présentant :
   - un plateau susceptible de prendre appui sur une surface de coupe tibiale,
   - un coulisseau susceptible d'être déplacé sur un moyen de glissière dans une direction sensiblement perpendiculaire audit plateau et présentant des moyens de solidarisation temporaire à une pièce ancillaire, comprenant une tige centro-médullaire et une plaque et susceptible de recevoir des moyens de calage d'épaisseur variable, préalablement mise en place à l'extrémité du fémur lorsque le genou est en flexion à environ 90°,
   - et un moyen moteur tel que, par exemple, un système vis et écrou, permettant au chirurgien de déplacer le coulisseau et de mettre le genou en tension lorsque le plateau est appliqué sur la coupe tibiale et la pièce ancillaire solidarisé audit coulisseau,
      un guide de perçage susceptible d'être monté sur ledit moyen de glissière au coulisseau et présentant
   - des trous de perçage permettant la mise en place ultérieure sur le fémur d'un bloc de coupe permettant d'assurer notamment, la coupe fémorale postérieure,
   - ledit guide pouvant, de préférence, comprendre ou être associé à un moyen de palpation de la partie antérieure du fémur, pour positionner le guide en alignement avec cette partie antérieure
   et des moyens de repérage déterminant la position dudit coulisseau et/ou dudit guide par rapport au plateau de la pièce mise sous tension, et donc de déterminer l'espace interarticulaire disponible en flexion.

Le dispositif permet ainsi, soit de déterminer la position du plan de coupe fémorale distale par détermination de la différence entre l'écart en extension et l'espace en flexion, soit, en cas de coupe distale effectuée d'emblée, de déterminer la position du plan de coupe fémorale postérieure pour obtenir une égalité sensible entre l'écart en extension et l'espace en flexion.

De façon avantageuse, le guide de perçage peut exister sous forme d'un jeu de plusieurs guides de dimensions différentes permettant au chirurgien d'adapter un guide correspondant à la taille du fémur, de façon à pouvoir déterminer un plan coupe postérieur optimal, et notamment n'entraînant pas de résection osseuse importante.

Le dispositif selon l'invention peut encore comprendre une pièce d'estimation de taille susceptible d'être montée sur ledit moyen de glissière pour pouvoir estimer, grâce à un moyen de repérage, la taille du fémur et permettre un bon choix de la pièce guide de perçage. A cet effet la dimension du plateau peut être avantageusement telle que l'extrémité fémorale puisse être prise entre ledit plateau et ladite pièce, à la façon d'un pied à coulisse.

Le dispositif peut encore comprendre un support de guide de coupe distale présentant un corps susceptible de coulisser sur le moyen de glissière, à partir duquel s'étend un bras qui s'étendra parallèlement à l'axe du genou en flexion, avec des moyens, par exemple un crantage, et/ou une graduation de repérage, pour recevoir et fixer en un emplacement précis le guide de coupe distale, ledit emplacement précis étant, de préférence, déterminé par le calcul de la différence entre l'espace en flexion et l'écart en extension.

Le dispositif selon l'invention peut notamment exister sous forme d'un jeu comprenant, ladite pièce de mise sous tension, ledit guide de perçage et, éventuellement, ladite pièce d'estimation de taille du fémur.

A ce jeu peut avantageusement être adjoint la pièce ancillaire ou un jeu de telles pièces, avec des moyens de calage pour déterminer l'écart articulaire en extension.

Ce dispositif d'écart en extension est avantageusement décrit dans les demandes WO98/25526 et US 319709 incorporées ici par référence.

La pièce ancillaire comprenant une tige centro-médullaire et une plaque tibiale et susceptible de recevoir une cale présente avantageusement des moyens lui permettant à la fois d'être positionnée de façon précise sur le coulisseau et d'être fixée temporairement sur celui-ci. Ces moyens peuvent par exemple comporter des encoches de positionnement et une fente permettant le passage d'une fixation, par exemple une fixation à baïonnette serrant la plaque de la pièce ancillaire contre le coulisseau.

Dans une forme de réalisation particulière de la pièce de mise sous tension, celle-ci comprend, s'étendant à partir dudit plateau, des moyens de glissière sur ou
dans lesquels est susceptible de coulisser ledit coulisseau, ce coulisseau étant de préférence déplaçable par une vis axialement immobilisée mais susceptible de tourner par rapport audit moyen de glissière et coopérant avec un écrou ou un taraudage correspondant du coulisseau pour permettre au chirurgien, en actionnant l'extrémité de la vis éloignée du plateau, de faire coulisser le coulisseau et mettre le genou en tension.

De préférence, lesdits moyens de glissière présentent une glissière interne dans laquelle est guidée le coulisseau, de façon que la surface externe desdits moyens de glissière permette le guidage dudit guide de perçage et d'autres du dispositif, ledit coulisseau présentant une partie, par exemple un relief permettant d'entraîner le guide du perçage sur le dispositif.

De préférence ledit guide de perçage est agencé pour recevoir un bras de palpage susceptible de s'appliquer sur la surface antérieure de l'extrémité fémorale pour limiter l'enfoncement dudit guide sur le moyen de guidage, pour déterminer la position de perçage.

En variante, le choix d'un guide de perçage de dimensions convenables et son positionnement précis sur le coulisseau en tension, peut assurer cette fonction de repérage du perçage.

De façon avantageuse, le coulisseau présente également des reliefs permettant un positionnement précis, par rapport au coulisseau, de la plaque de la pièce ancillaire et un moyen de fixation rapide, par exemple du type à baïonnette, permettant de solidariser temporairement ladite tige par rapport au coulisseau dans la position dans laquelle le plateau de la tige a été reçu sur le coulisseau.

De façon avantageuse, la pièce d'estimation de taille pour palper l'extrémité antérieure du fémur peut être formé d'une pièce avec un corps susceptible de coulisser sur ou dans le moyen de glissière, ce corps coulissant présentant un bras transversal de palpage, de préférence articulé autour d'un axe parallèle à l'axe de coulissement.

Le bras de palpage peut comporter, le cas échéant, une tige déplaçable tant longitudinalement que transversalement par rapport audit bras.

L'invention a également pour objet des procédés de pose de prothèse totale de genou mettant en oeuvre le dispositif selon l'invention.

Dans un mode de mise en oeuvre, un tel procédé peut comporter les étapes suivantes :
- après résection de l'extrémité articulaire proximale par coupe tibiale au niveau du plateau tibial, mise en place d'une pièce ancillaire, comprenant une tige centro-médullaire et une plaque tibiale et susceptible de recevoir une cale, dans le canal médullaire fémoral après mise en flexion du genou, de sorte que la plaque de ladite pièce ancillaire vienne s'appliquer contre la face distale des condyles fémoraux,
- après remise en extension, équilibrage du genou en extension, tel que décrit par exemple dans le document WO98/25526 et mesure de l'écart en extension, par exemple au moyen des cales décrites dans ledit document ; ceci détermine une mesure appelée écart en extension,
- après mise en flexion du genou, mise en place de la pièce de mise sous tension par fixation du coulisseau contre ladite plaque de pièce ancillaire puis, éventuellement, estimation de la taille du fémur en utilisant la pièce d'estimation de taille susceptible d'être montée de façon coulissante sur ladite pièce de mise sous tension, le fémur en flexion étant pris entre le plateau de ladite pièce de mise sous tension et le moyen de palpage de ladite potence montée sur la pièce mise sous tension,
- après éventuelle dépose de cette pièce d'estimation, mise en tension ligamentaire du genou en position de flexion, le plateau de ladite pièce de tension venant prendre appui sur le plateau de coupe tibiale et le moyen de déplacement du coulisseau étant actionné jusqu'à obtention de la tension convenable, le fémur pouvant librement tourner en rotation autour de la tige centreo-médullaire de la pièce ancillaire pendant le déplacement de mise sous tension pour un parfait équilibrage ligamentaire,
- mise en place du guide de perçage de la taille estimée précédemment.
- Confirmation de la taille à l'aide du bras de palpage antérieur et lecture de l'espace en flexion virtuel, par exemple par lecture d'une graduation sur la pièce de mise en tension, par exemple graduée sur sa glissière, pour contrôler la position du guide de perçage sur le dispositif de mise sous tension.
- Perçage des deux trous du guide de coupe, une fois l'optimisation de la balance antéro-postérieure effectuée.
- mise en place d'un guide de coupe distale, par exemple par vissage sur la face antérieure du fémur, puis réglage du plan de coupe distale après calcul de la distance de réglage de coupe distale égale à la différence entre l'espace en flexion et l'écart en extension, puis fixation définitive du guide de coupe distale à l'aide de broches sur la partie antérieure du fémur.
- dépose de la pièce de mise en extension puis de la pièce ancillaire.
- réalisation de la coupe distale à l'aide du guide de coupe distale puis dépose du guide de coupe distale,
- mise en place d'un bloc de coupe grâce aux trous réalisés à l'aide de la pièce de guidage de perçage puis réalisation à l'aide de ce bloc de coupe des différentes autres coupes à savoir la coupe postérieure, la coupe antérieure, et les deux chanfreins associés.

Dans un autre mode de mise en oeuvre d'un procédé selon l'invention, le chirurgien commence par mettre en place le guide de coupe distale et règle, manuellement, la position de la coupe distale ; après fixation par broches ou vis de guide de coupe distale, il pratique la coupe distale :
- il procède ensuite au contrôle de l'écart en extension à l'aide des moyens de calage ou de mise en extension classiques ;
- puis mise en place de la pièce ancillaire comme dans le mode de mise en oeuvre précédent ;
- mise en place, sur le plateau de la tige de la pièce de mise sous tension et éventuellement estimation de la taille du fémur selon le mode de mise en oeuvre précédent,
- mise en tension ligamentaire du genou fléchi provoquant la rotation éventuelle du fémur pour l'adaptation à la bonne tension ligamentaire, puis blocage du dispositif de mise sous tension dans sa position de tension,
- mise en place du guide de perçage de la taille estimée précédemment et confirmation ou modification de cette taille,
- lecture de l'espace virtuel en flexion sur le dispositif de mise sous tension, ces opérations étant analogues ou identiques à celles de l'étape correspondante du procédé précédent,
- positionnement du guide de perçage pour que l'espace en flexion reproduise, de préférence à l'identique, l'écart en extension qui a été imposé par la coupe distale initiale fémorale,
- perçage des deux trous destinés à fixer le guide de coupe
- dépose du guide de perçage, du dispositif de mise sous tension puis de la pièce ancillaire.
- mise en position du bloc de coupe dans les trous réalisés à l'aide du guide de perçage puis réalisation des différentes coupes conformément à l'étape finale du procédé précédent.

D'autres avantages et caractéristiques de l'invention apparaîtront à la lecture de la description suivante, faite à titre d'exemple non limitatif et se référant au dessin annexé dans lequel :

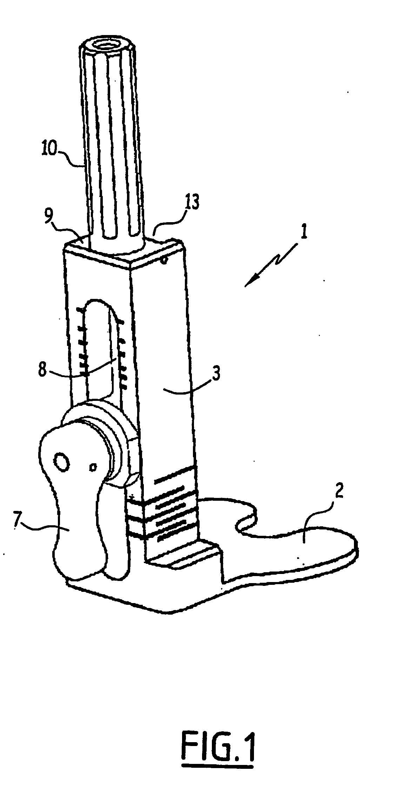

la figure 1 représente une vue en perspective de la pièce de mise sous tension.

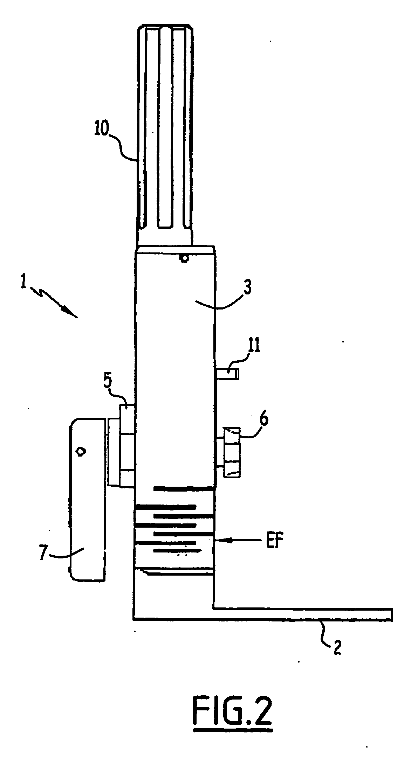

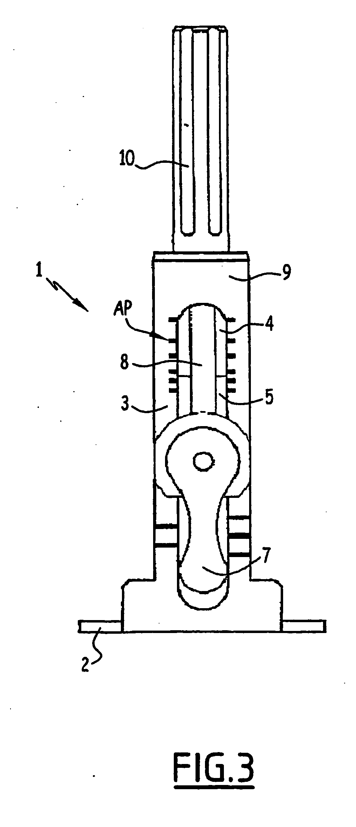

les figures 2, 3 et 4 représentent des vues en élévation, de profil et de dessus de ce dispositif,
la figure 5 représente une vue en perspective d'une une pièce ancillaire comprenant une tige centro-médullaire et une plaque tibiale et susceptible de recevoir une cale.
les figures 6 à 9 représentent des vues en élévation, de profil, supérieure et inférieure de cette tige.
la figure 10 représente une vue en perspective d'un gabarit de perçage,
les figures 11, 12 et 13 représentent des vues en élévation, de profil et de dessus de cette pièce de guide de perçage,
la figure 14 représente une vue en perspective d'une pièce d'estimation de taille,
les figures 15, 16, 17 et 18 représentent des vues en élévation, de profil et de dessus de cette pièce,
la figure 19 représente une vue en perspective d'un support de guide de coupe distale,
les figures 20 à 23 représentent des vues en élévation, de profil et de dessus de cette pièce,
les figures 24 à 32 représentent des vues schématiques en perspective de différents stades d'une opération de pose de prothèse de genou mettant en oeuvre le dispositif selon l'invention,
les figures 33 à 41 représentent des vues des différents stades d'une telle pose de prothèse dans un autre mode de mise en oeuvre de l'invention.

Le dispositif selon l'invention comporte un dispositif de mise en extension du genou 1. Celui-ci comprend à partir d'un plateau 2, un double montant 3 perpendiculaire au plateau et déterminant une fente intérieure 4 dans la même direction, dont les faces forment un moyen de glissière pour guider en translation un coulisseau 5.

Le coulisseau 5 présente un moyen de fixation à baïonnette 6, du côté du montant 3 regardant vers le plateau 2, ce moyen de fixation à baïonnette étant actionné, par l'intermédiaire d'une tige traversant le coulisseau, un levier 7 permettant de tourner le moyen de fixation à baïonnette 6. A sa face supérieure, le coulisseau 5 se poursuit d'un seul tenant par une tige filetée 8 qui traverse librement l'extrémité supérieure 9 du montant et pénètre dans le taraudage interne d'un écrou 10 monté libre en rotation, mais immobile en translation, sur l'extrémité 9 du montant ; ledit écrou 10 présentant une forme allongée vers le haut avec des striures pour former une mollette d'actionnement. Enfin, l'extrémité supérieure du coulisseau 5, du côté regardant vers la base 2, présente un ergot transversal 11 qui émerge hors du montant 3.

On comprend donc qu'en tournant la mollette-écrou 10, dans un sens ou dans un autre, on fait monter ou descendre, par rapport au montant 3, la tige filetée 8, et par conséquent le coulisseau 5 guidé dans le moyen de glissière interne formé par ledit montant 3. Il en résulte que si une pièce est fixée contre le coulisseau par le biais de la fixation à baïonnette 6, ou si une pièce repose sur l'ergot 11, cette pièce suivra les mouvements du coulisseau.

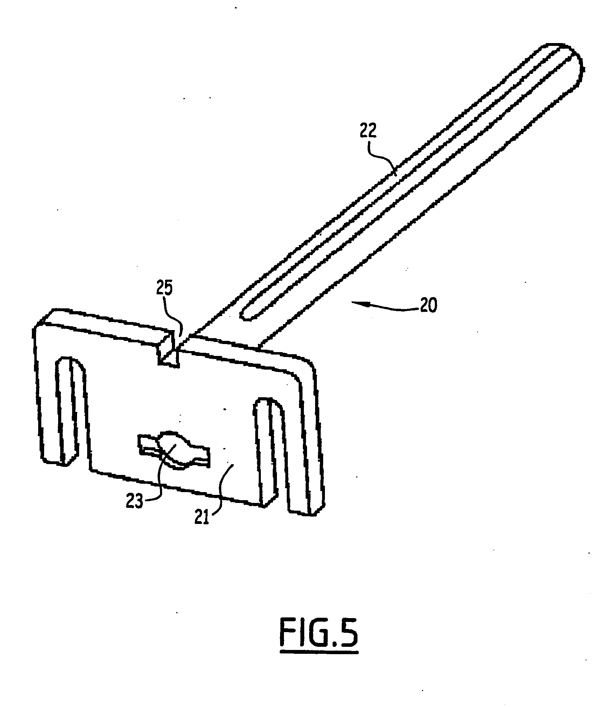

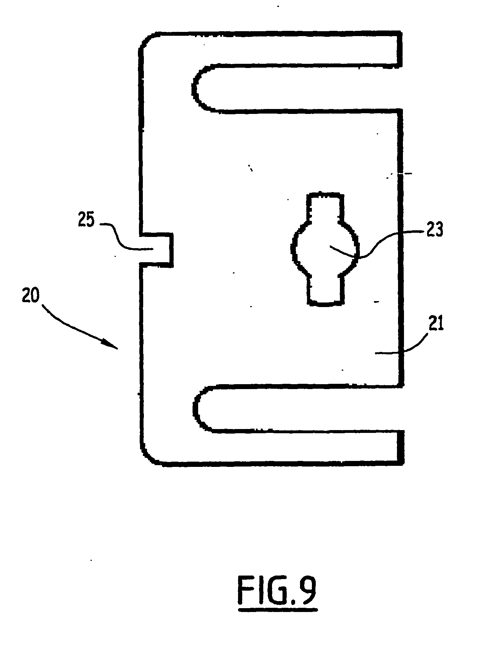

Le dispositif représenté est destiné à coopérer avec une pièce ancillaire selon les demandes WO98/25526 et US 319709 et on se réfèrera à ce sujet aux figures 5 à 9.

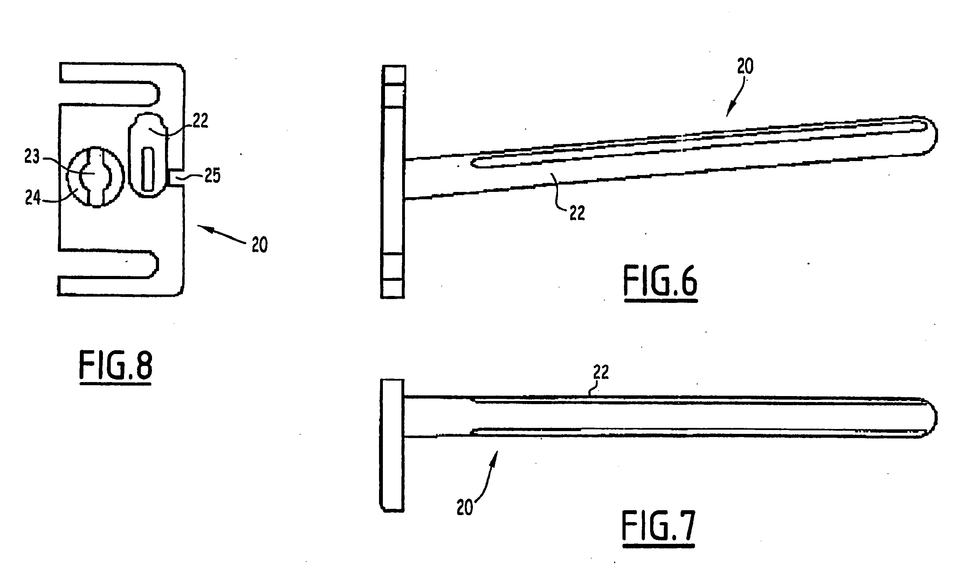

Cette pièce comporte une embase ou plaque 21 qui présente sur l'une de ses faces, et vers un bord antérieur d'un point de vue anatomique pour le fémur, une tige centro-médullaire proprement dite 22 qui, dans un plan sagittal, comme le montre la figure 7, est sensiblement perpendiculaire à l'embase 21, mais qui, dans un plan frontal, comme le montre la figure 6, présente par rapport à la normale à l'embase une inclinaison correspondant au valgus fémoral normal.

Cette pièce auxiliaire est destinée à permettre la recherche d'un équilibrage ligamentaire en extension, grâce aux moyens de calage et à la mise en oeuvre éventuelle de moyens de distraction en extension.

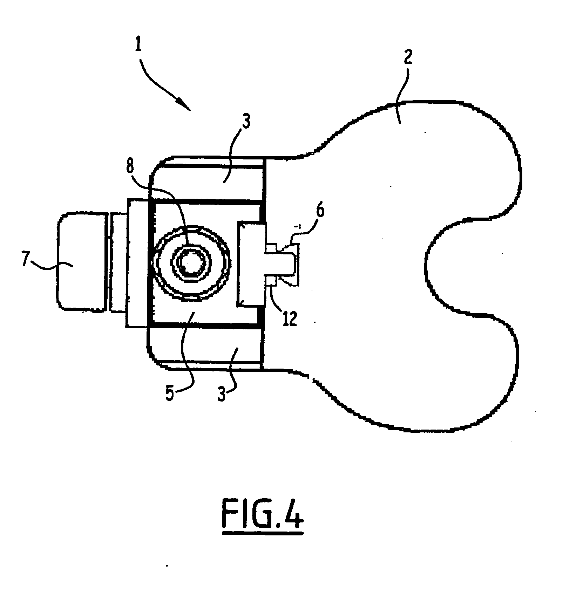

On voit que, en alignement dans un plan sagittal avec l'extrémité inférieure de la tige 22, l'embase 21 présente un passage oblong 23 présentant un trou central se poursuivant de part et d'autre par des encoches diamétralement opposées et destinées à permettre le passage de la tête de baïonnette 6. Cette forme est réalisée par le fond, disposé du côté inférieur de l'embase, d'un lamage circulaire 24, de sorte que l'on comprend que lorsque la tête de fixation 6 est introduite, par le côté inférieur visible sur la figure 5, dans le passage 23, une rondelle 12 située sur le coulisseau sous la tête de baïonnette 6 pénètre dans le lamage circulaire 24 pour un positionnement précis de forme du coulisseau, et donc de la pièce 1, par rapport à la pièce 20. La tête de baïonnette 6, qui dépasse sur l'extrémité supérieure de l'embase 21, et qui est conformée en forme de rampe, comme on le voit sur la figure 4, vient définitivement bloquer l'embase 21 contre le coulisseau, tandis que l'ergot 11 pénètre dans une encoche correspondante 25 de l'embase 21 pour parfaire le blocage de forme réciproque des deux parties.

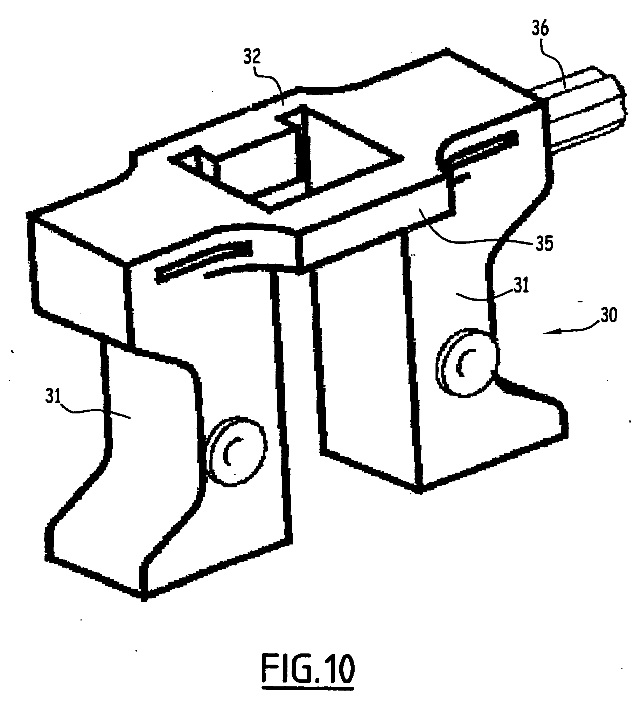

La pièce ancillaire 20 étant ainsi bloquée sur le coulisseau, on peut venir mettre en place un gabarit ou guide de perçage et on se référera à ce sujet aux figures 10 à 13.

Ce gabarit présente une forme générale en U avec deux jambes 31 et une base 32, cette base 32 présentant un passage central de forme rectangulaire 33 prolongeant l'espace vide entre les deux jambes 31 et présentant un relief interne 34.

Le gabarit de perçage peut ainsi être monté coulissant sur le montant 3 servant de moyen de glissière externe pour les deux jambes 31 et le trou 33, un positionnement précis étant en outre assuré par l'introduction du relief 34 par l'échancrure 13 laissée, dans le montant, du côté regardant vers la base 2.

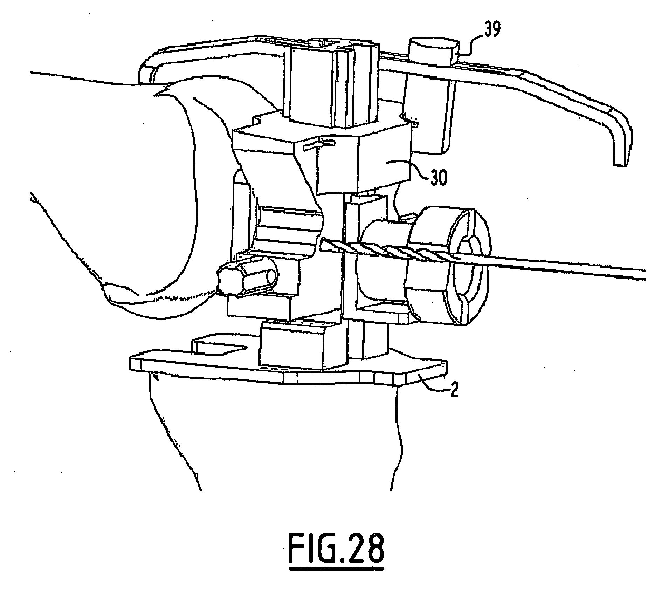

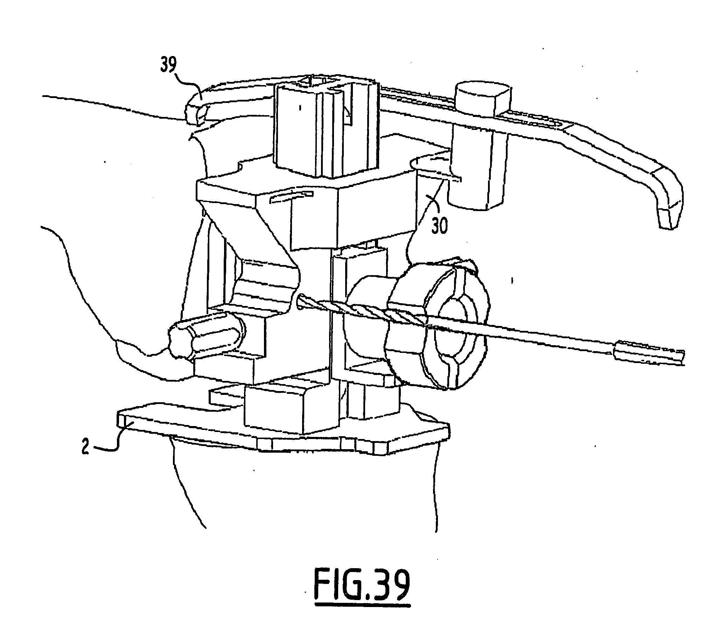

Le gabarit présente encore des fentes 38 permettant la mise en place d'un bras palpeur 39, vu par exemple sur les figures 28 et 39, qui permet de déterminer précisément la position du gabarit 30 par rapport au bord antérieur du fémur.

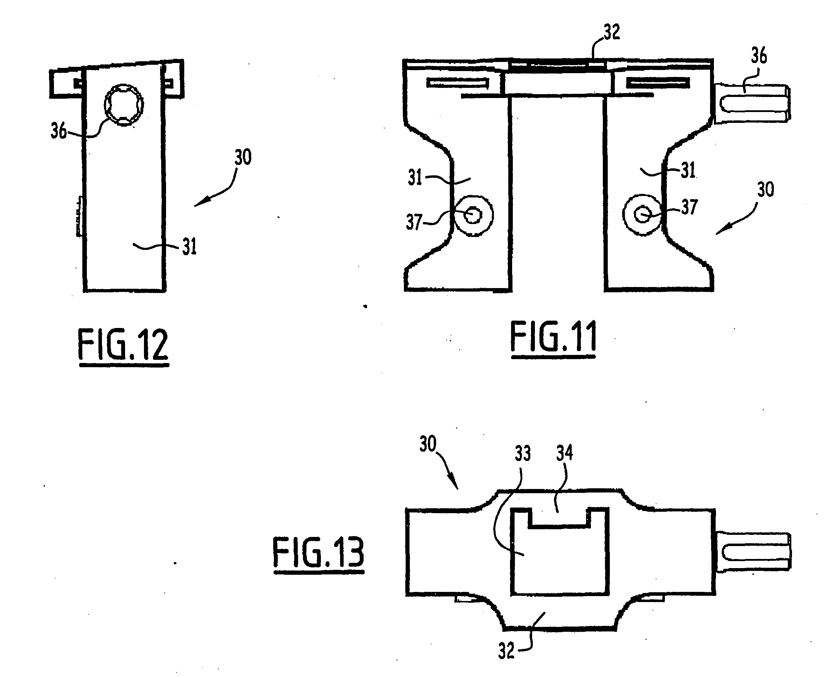

Comme on le voit sur la figure 12, notamment, la surface supérieure du gabarit de perçage 30 est inclinée selon un plan qui se dirige vers la tige 22 lorsque le gabarit de perçage 30 est mis en place d'une façon telle que un rebord 35 du gabarit de perçage vienne s'appliquer contre le bord de l'embase 21 qui porte l'encoche 25 lorsque celle-ci est également en place sur le coulisseau du dispositif, ce plan incliné étant destiné à visualiser le plan de coupe fémoral antérieur.

Un moyen de serrage à vis 36 permet d'immobiliser le guide de perçage par rapport au montant 3, l'extrémité (non représentée) de la vis 36 venant s'appliquer contre ce montant.

Deux trous 37, traversant les jambes 31, servent de guide pour des broches de perçage pour forer dans l'extrémité du fémur, les trous de fixation d'un guide de coupe usuel.

Les faces inférieures des jambes 31 se déplacent au regard d'une graduation EF portée par la glissière 3 pour former l'index de lecture de l'espace en flexion sur cette graduation.

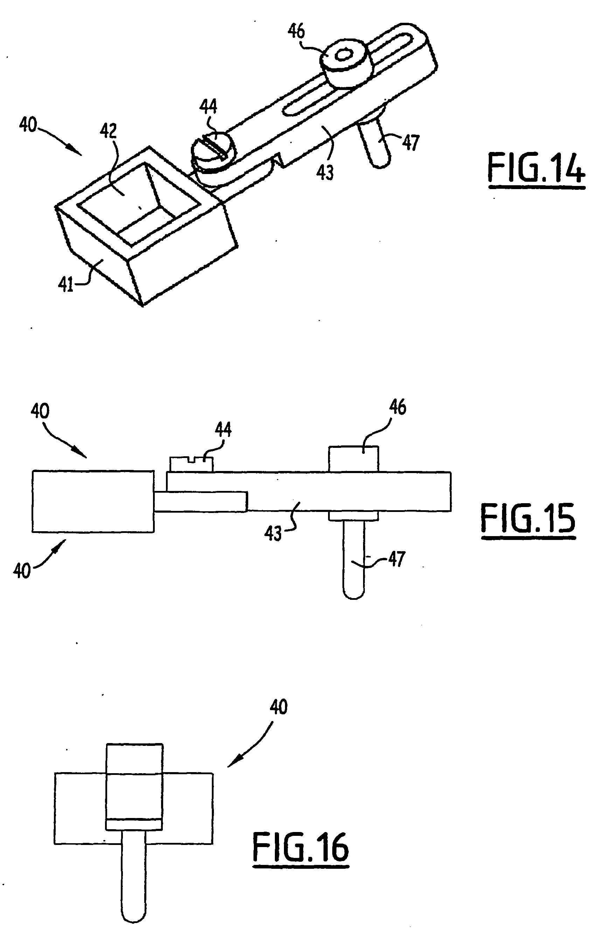

Le dispositif de mise sous tension 1 peut encore recevoir une pièce d'estimation de taille de fémur 40, qui est décrite à l'occasion des figures 14 à 18.

Ce dispositif présente un corps carré 41 muni d'un passage central carré 42 permettant un glissement précis sur les surfaces extérieures du montant 3. Du côté orienté vers l'embase 2, ce corps présente un bras 43 qui présente, de préférence, une articulation 44, la partie la plus longue de ce bras 43 formant elle-même un glissière 45 dans laquelle peut coulisser le support 46, d'une pointe de palpage 47 et l'on comprend donc que l'on peut de cette façon, venir disposer la pointe de palpage 47 à l'emplacement dans lequel le fémur présente son encombrement antérieur maximal.

Grâce à ce bras palpeur, on peut utiliser l'ensemble formé par le dispositif de mise sous tension 1 et la pièce 40 montée coulissante sur le montant 3, comme un pied à coulisse lorsque le plateau 2 vient s'appliquer contre la partie postérieure de l'extrémité du fémur, alors que le palpeur 47 vient s'appliquer contre la partie antérieure de cette extrémité fémorale. La dimension antêro-postêrieure d'extrémité fémorale peut ainsi être déterminée par lecture de la position du bord inférieur 48 du corps 41 sur la graduation AP que l'on voit sur le montant 3, notamment figure 5, du côté présentant le levier 7.

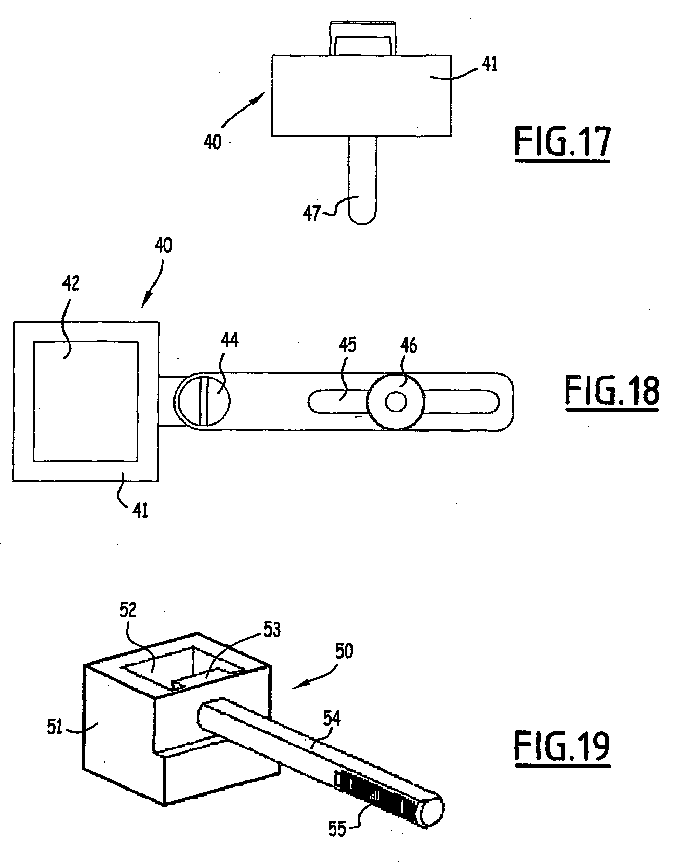

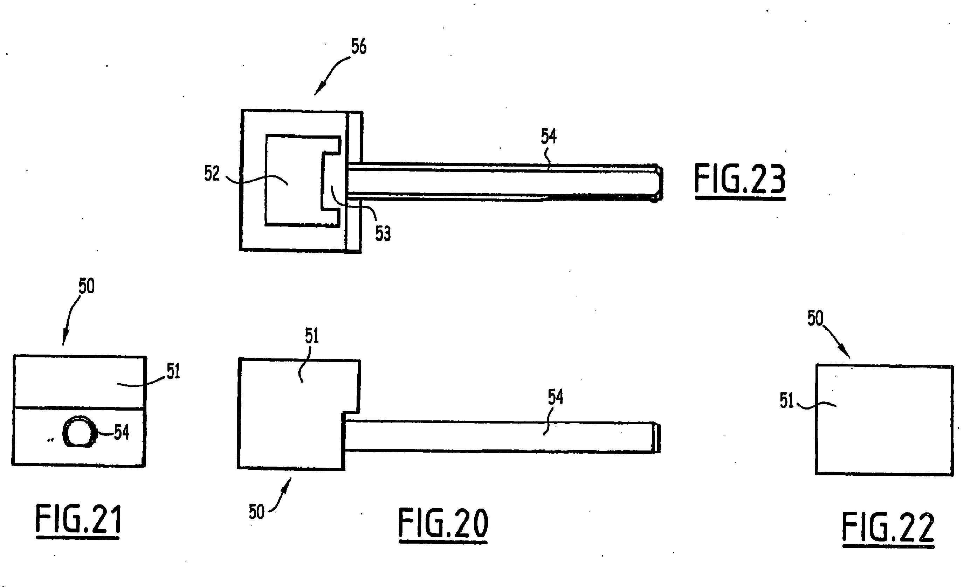

Le dispositif selon l'invention peut encore avantageusement comporter un support de guide de coupe distale 50 que l'on retrouve sur les figures 19 à 23. Le support comporte un corps 51 muni d'un passage carré 52 et présentant un relief 53 destiné à coopérer avec la partie échancrée 13 du montant 3, de façon que le coulissement du corps 50 sur le montant 3 se fasse avec une grande précision. Du côté de ce relief 53 et donc du côté de la base 2, ce corps présente une tige perpendiculaire 54 sur lequel on peut venir enfiler un guide de coupe distale que l'on peut ainsi positionner correctement du côté antérieur de l'extrémité fémorale à la distance souhaitable du montant 3, et donc du plan de référence déterminé par la face proximale de la plaque 21 de la pièce auxiliaire 20.

A cette fin, le bras 54 présente un crantage 55 permettant une parfaite immobilisation du guide de coupe distale sur le bras 54, ledit bras présentant une graduation (non représentée) référencée par rapport à ladite face proximale de la plaque 21 pour positionner le guide de coupe distale à la distance égale à la différence entre l'espace en flexion et l'écart en extension.

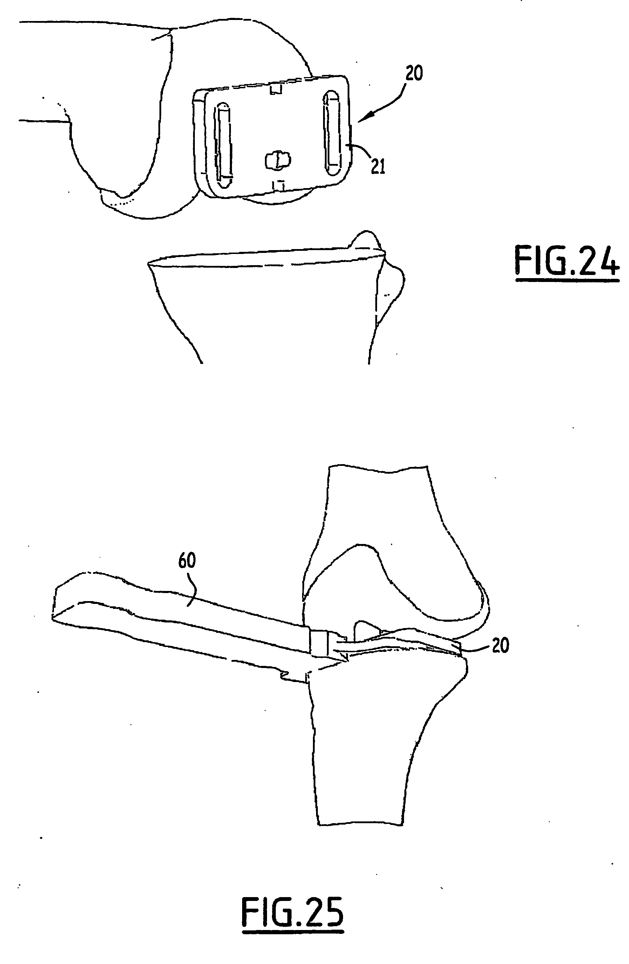

Un procédé mettant en oeuvre le dispositif est maintenant décrit à l'occasion des figures 24 à 32.

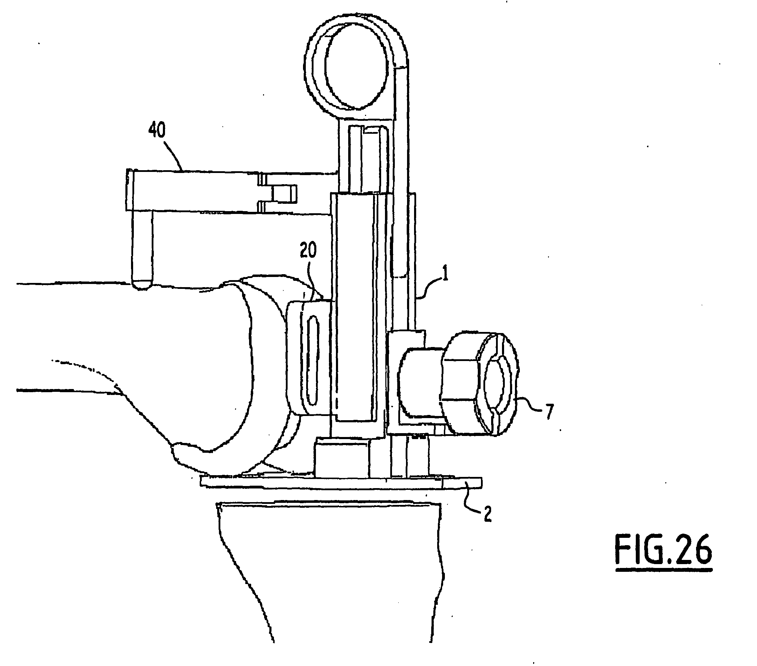

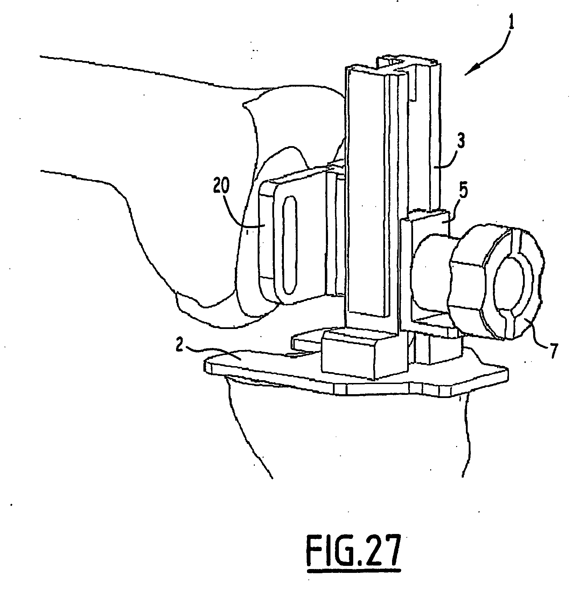

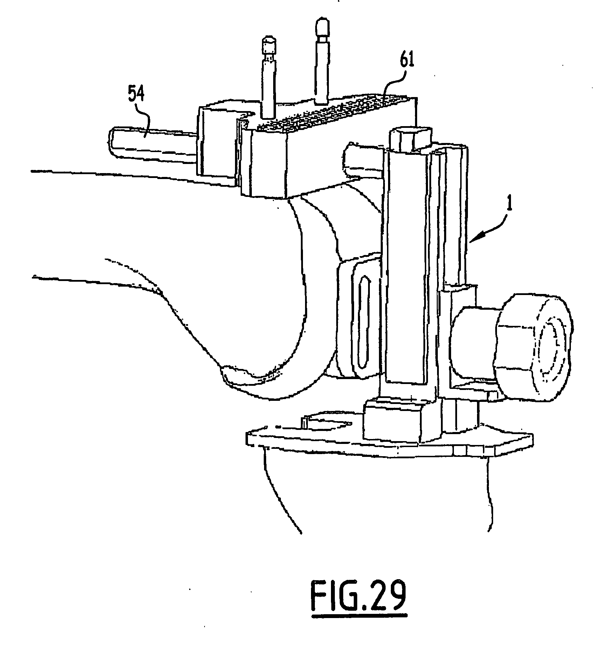

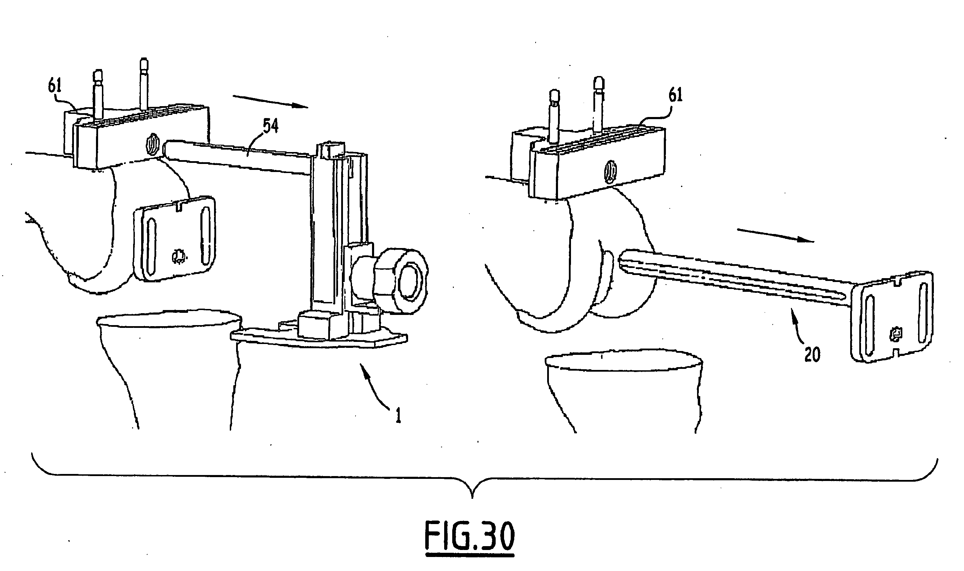

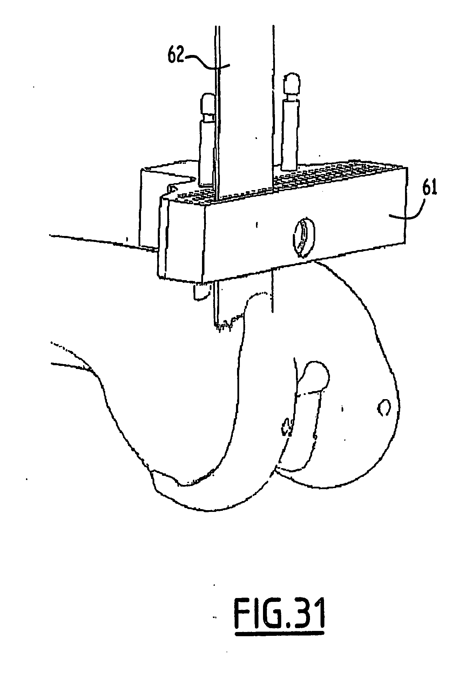

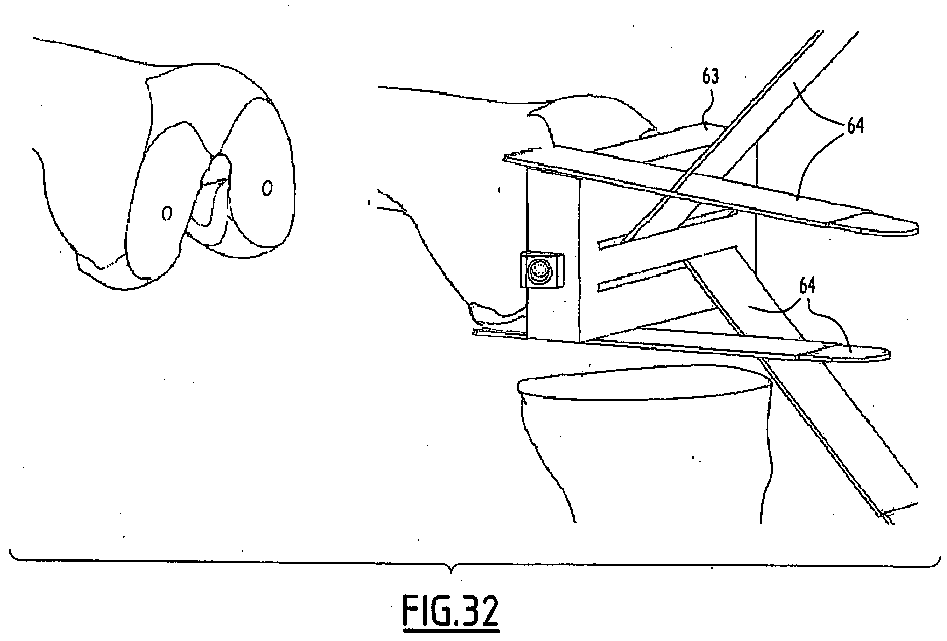

Les gestes de ce procédé sont les suivants :
1. (figure 24) mise en place de la pièce ancillaire 20 la tige 22 pénétrant dans le canal médullaire et l'embase étant appliquée par sa face supérieure contre l'extrémité inférieure des condyles fémoraux ;
   Cette opération s'effectue le genou fléchi.
2. (figure 25) après remise du genou en extension, on procède à la mise sous tension des ligaments en écartant le plateau de coupe tibiale de l'extrémité fémorale, à l'aide d'un dispositif distracteur 60 tel que les moyens de calage connus, pour obtenir un écart d'extension tibio-fémoral rectangulaire, dans lequel les axes du tibia et du fémur sont correctement positionnés, cet écart en extension étant alors lu par le chirurgien de la façon connue;
3. (figure 26) après avoir remis le genou en flexion, on fixe le dispositif 1 comme représenté sur la figure 26, contre la face inférieure de l'embase 21 par le moyen de fixation à baïonnette et l'on tourne le bouton ou mollette 10 jusqu'à amener le plateau 2 au contact de l'extrémité postérieure des condyles fémoraux.
   On peut alors mettre en place sur le montant 3 le - dispositif d'estimation de taille 40 et lire sur la graduation AP la dimension antéro-postérieure de cette extrémité fémorale à la manière d'un pied à coulisse ;
   Sur la figure 26 le dispositif 40 présente des moyens de montage et de repérage qui forment une variante de ceux représentés sur la figure 14.
4. après dépose de la pièce 40 et comme on le voit sur la figure 27, on met le genou en tension ligamentaire en tournant le levier 7, la base 2 prenant appui sur le plateau de coupe tibiale. Ce faisant le fémur peut tourner librement autour de la tige 22 située dans son canal médullaire et prendre la position adéquate jusqu'à obtention du degré de tension des ligaments souhaités par le chirurgien ;
5. (figure 28) le dispositif étant bloqué dans cette position, on met en place sur le montant 3, le gabarit de perçage 30, muni du bras palpeur 39 pour confirmer la taille antéro-postérieure de l'extrémité du fémur. En cas de besoin on remplace le gabarit de perçage par un autre gabarit de dimension différente pour optimiser la dimension et l'espace en flexion lu sur la graduation EF ;
   lorsque le gabarit est estimé être dans la bonne position, on fait passer deux broches de perçage par ses deux trous 37, on fore les trous, dans l'extrémité des condyles fémoraux, qui serviront de guide pour la mise en place du guide de coupe.
   A ce moment, le chirurgien procède à la lecture de l'espace en flexion en regardant la position de l'extrémité inférieure du gabarit 30 sur l'échelle EF fixée sur le montant 3 ;
6. (figure 29) après avoir noté cet espace, on dépose le gabarit de perçage et on met en place sur le montant 3 le support de guide de coupe distale 50 sur le bras duquel on enfile le guide de coupe distale 61. Ce guide de coupe distale 61 est placé sur ce bras à une distance égale à la différence entre l'espace en flexion qui avait été lu sur la graduation EF et l'écart en extension qui avait été initialement lu dans la phase 2.
   Le gabarit étant maintenu dans cette position le long du bras 54, le chirurgien fixe le gabarit par des broches vissées dans la partie antérieure de l'extrémité fémorale.
7. (figure 30) on procède ensuite à la dépose de l'ensemble pièce de mise sous tension 1 et support guide de coupe 50, puis à l'extraction de la pièce ancillaire 20 ;
8. (figure 31) on peut alors procéder à la réalisation de la coupe distale d'une façon classique dans la position qui a été définie par le guide de coupe distale 61 qui guide l'outil de coupe 62 ;
9. après dépose du guide de coupe distale 61, on met en place le bloc de coupe 63 dans les perçages réalisés lors de la phase 5, jusqu'à appliquer le bloc de coupe et à l'immobiliser contre la coupe distale du fémur réalisée à l'étape 8 (figure 32). Le chirurgien peut alors procéder avec les outils 64 aux différentes autres coupes et notamment à la coupe postérieure, le guide de coupe étant agencé dans ses dimensions par rapport aux repères déterminés par les perçages, pour enlever la quantité optimale du bord postérieur fémoral. I1 procède également aux éventuelles coupes antérieures et aux coupes en biseau de l'extrémité fémorale, s'il y a lieu.

Le chirurgien peut alors, après dépose des blocs de coupe, poursuivre la procédure et poser les pièces de la prothèse complète de genou.

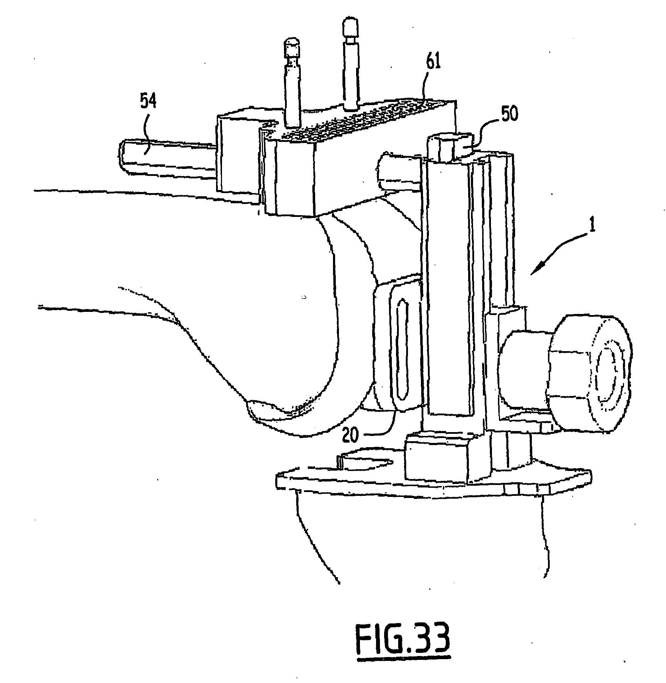

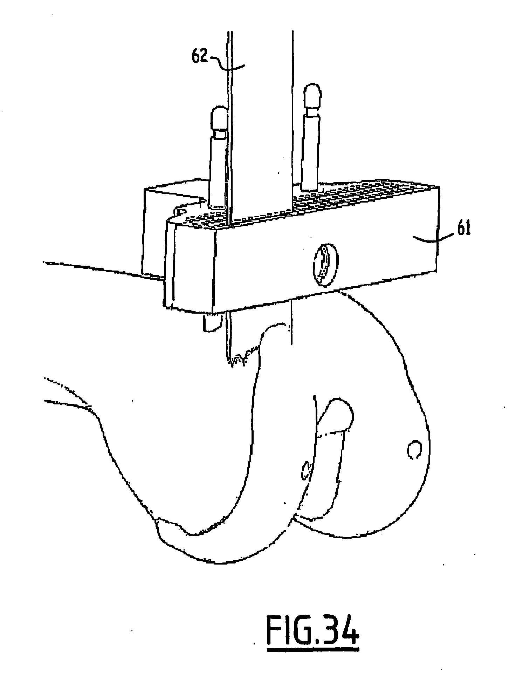

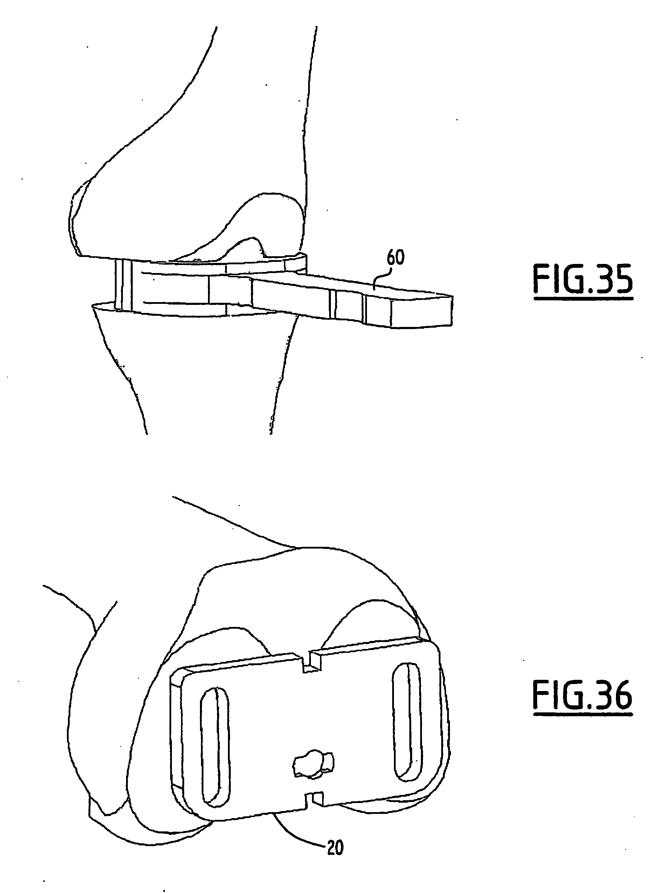

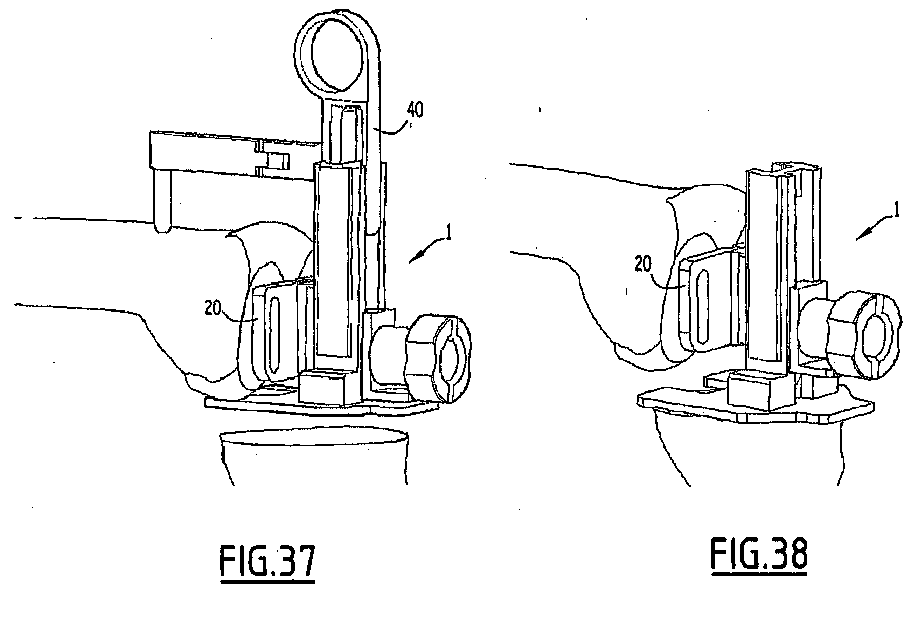

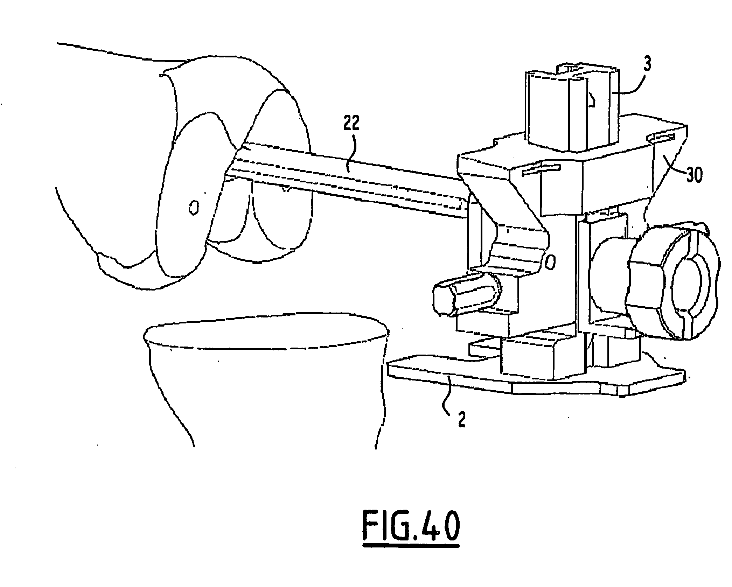

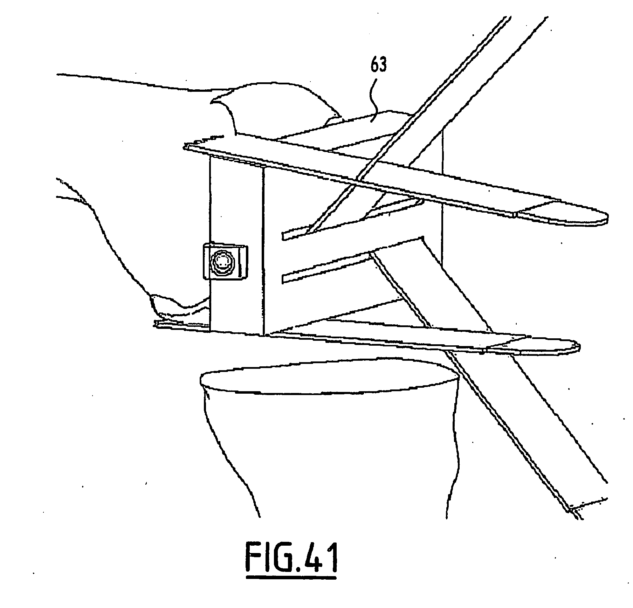

Le dispositif selon l'invention peut également être utilisé dans une variante de procédé dans lequel, comme cela est souvent le cas, le chirurgien procède initialement à la coupe distale, comme décrit sur les figures 33 à 41. Ce procédé peut comporter les gestes suivants :
1. sur le fémur en flexion dans lequel il a introduit la tige de la pièce ancillaire 20 puis fixé la pièce 1, dans une position dans laquelle sa base prend appui sur le plateau de coupe tibiale, le chirurgien pose le support-guide de coupe distale 50, puis positionne sur celui-ci, à l'emplacement qu'il juge optimal, le guide de coupe distale qu'il fixe sur le bord antérieur du fémur par des broches (figure 33) ;
2. après retrait des pièces 1, 20 et 50, il réalise la coupe distale (figure 34) ;
3. après avoir remis le genou en extension comme le montre la figure 35, il contrôle par des moyens usuels 60 l'écart en extension entre les deux plans tibial et fémoral ainsi réalisés ;
4. après remise du genou en flexion, il met en place la pièce ancillaire selon la figure 36 ;
5. comme on le voit sur la figure 37, il remet en place la pièce de mise sous tension 1 et, avec la pièce 40, il mesure la dimension antéro-postérieure du fémur à la façon d'un pied à coulisse ;
6. après dépose de la pièce 50, comme on le voit sur la figure 38, il procède à la mise en tension ligamentaire, ce qui provoque l'adaptation du fémur par rotation autour de la tige 22, et ceci jusqu'au blocage en tension de la pièce de mise sous tension 1 ;
7.ensuite mise en place du guide de perçage 11, confirmation de la taille fémorale à l'aide d'un bras palpeur présenté par cette pièce comme le montre la figure 39 ; après confirmation de la taille fémorale et éventuellement remplacement du gabarit de perçage par un autre d'une taille différente, recherche d'une position
   dans laquelle l'espace en flexion sur la graduation EF est égal à l'écart en extension, puis perçage des trous de fixation du guide du bloc de coupe par des broches vissées à travers les trous 37 ; on peut alors vérifier, sur la graduation EF, l'espace en flexion qui a été obtenu sur l'échelle EF.
8. comme le montre la figure 40, il procède ensuite au dépôt de l'ensemble des pièces qui ont été fixées ;
9. il peut alors, en utilisant les perçages qui viennent d'être réalisés, mettre en place le bloc de coupe comme le montre la figure 41 et procéder aux différentes coupes, dont la coupe du bord postérieur de l'extrémité fémorale.

## Revendications

1. Dispositif pour pose d'une prothèse totale du genou comportant :
une pièce de mise sous tension (1) présentant :
- un plateau (2) susceptible de prendre appui sur une surface de coupe tibiale,
- un coulisseau (5) susceptible d'être déplacé sur un moyen de glissière (3) dans une direction sensiblement perpendiculaire audit plateau (2) et présentant des moyens (6, 11) de solidarisation temporaire à une pièce ancillaire 20 comprenant une tige centro-médullaire et une plaque et susceptible de recevoir des moyens de calage d'épaisseur variable, préalablement mise en place à l'extrémité du fémur lorsque le genou est en flexion à environ 90°, et permettant d'obtenir un écart en extension articulaire.
- et un moyen moteur (8, 10) permettant au chirurgien de déplacer le coulisseau (5) et de mettre le genou en tension lorsque le plateau est appliqué sur la coupe tibiale et la pièce ancillaire 20 solidarisée audit coulisseau,
un guide de perçage (30) susceptible d'être monté sur ledit moyen de glissière (3) et présentant
- des trous de perçage (37) permettant la mise en place ultérieure sur le fémur d'un bloc de coupe permettant d'assurer les coupes fémorales postérieures,
- ledit guide (30) pouvant comprendre ou être associé à un moyen de palpation de la partie antérieure du fémur pour positionner le guide en alignement avec cette partie antérieure
- et des moyens de repérage (EF) déterminant la position dudit coulisseau (5) et/ou dudit guide (30) par rapport au plateau (2) de la pièce mise sous tension (1), et donc de déterminer l'espace interarticulaire disponible en flexion,
ledit dispositif permettant ainsi, soit de déterminer la position du plan de coupe fémorale distale par détermination de la différence entre l'écart en extension et l'espace en flexion, soit, en cas de coupe distale effectuée d'emblée, de déterminer la position du plan de coupe fémorale postérieure pour obtenir une égalité sensible entre l'écart en extension et l'espace en flexion.

2. Dispositif selon la revendication 1 **caractérisé en ce qu'**il comprend une pièce d'estimation de taille (40) -susceptible d'être montée sur ledit moyen de glissière (3) pour pouvoir estimer, grâce à un moyen de repérage, la taillé du fémur et permettre un bon choix de la pièce guide de perçage (30).

3. Dispositif selon la revendication 2 **caractérisé en ce que** la dimension du plateau (2) est telle que l'extrémité fémorale puisse être prise entre ledit plateau et ladite pièce (40), à la façon d'un pied à coulisse.

4. Dispositif selon l'une des revendications 1 à 3 **caractérisé en ce qu'**il comprend un support de guide de coupe distale (50) présentant un corps (51) susceptible de coulisser sur le moyen de glissière (3), à partir duquel s'étend un bras (54) qui s'étendra parallèlement à l'axe du genou en flexion, avec des moyens pour recevoir et fixer en un emplacement précis le guide de coupe distale, ledit emplacement précis étant susceptible d'être déterminé par le calcul de la différence entre l'espace en flexion et l'écart en extension.

5. Dispositif selon l'une des revendications 1 à 4 **caractérisé en ce que** la pièce de mise sous tension (1), comprend, s'étendant à partir dudit plateau (2), des moyens de glissière (3) sur ou dans lesquels est susceptible de coulisser ledit coulisseau (5), ce coulisseau (5) étant déplaçable par un ensemble vis (8) écrou (10) pour permettre au chirurgien, de faire coulisser le coulisseau et mettre le genou en tension.

6. Dispositif selon la revendication 5 **caractérisé en ce que** lesdits moyens de glissière (3) présentent une glissière interne dans laquelle est guidée le coulisseau (5), et une surface externe permettant le guidage dudit guide de perçage (30) et d'autres pièces du dispositif, ledit coulisseau présentant une partie permettant d'entraîner le guide du perçage sur le dispositif.

7. Dispositif selon l'une des revendications 1 à 6 **caractérisé en ce que** le guide de perçage (30) est agencé pour recevoir un bras de palpage susceptible de s'appliquer sur la surface antérieure de l'extrémité fémorale pour limiter l'enfoncement dudit guide sur le moyen de guidage, pour optimiser la position de perçage.

8. Dispositif selon l'une des revendications 1 à 7 **caractérisé en ce que** le coulisseau (15) présente des reliefs (11) permettant un positionnement précis, par rapport au coulisseau, de la plaque (21) de la pièce ancillaire (20) et un moyen de fixation rapide (6) permettant de solidariser temporairement ladite plaque (21) par rapport au coulisseau (5)

9. Dispositif selon l'une des revendications 1 à 8 **caractérisé en ce que** la pièce d'estimation de taille (40) pour palper l'extrémité antérieure du fémur est formée d'une pièce avec un corps (41) susceptible de coulisser sur ou dans le moyen de glissière (3), ce corps coulissant présentant un bras transversal (43) de palpage articulé autour d'un axe (44) parallèle à l'axe de coulissement.

## Claims

1. Device for fitting a total knee prosthesis, comprising:
a tensioning element (1) having:
- a plate (2) capable of bearing on a tibial cutting surface,
- a slide (5) capable of being displaced on a sliding means (3) in a direction substantially perpendicular to said plate (2) and having means (6, 11) for temporarily connecting it to an ancillary element 20 which comprises a centromedullary rod and a plate and which is capable of receiving adjustment means of variable thickness which have previously been positioned at the end of the femur when the knee is at approximately 90° of flexion, and allowing an articular spacing in extension to be obtained,
- and a motor means (8, 10) allowing the surgeon to displace the slide (5) and place the knee under tension when the plate is brought to bear on the tibial cut and the ancillary element 20 is connected to said slide,
a drilling guide (30) capable of being mounted on said sliding means (3) and having
- drilled holes (37) which allow the subsequent positioning on the femur of a cutting assembly which allows the posterior femoral cuts to be carried out,
- it being possible for said guide (30) to comprise or be associated with means for palpating the anterior part of the femur in order to position the guide in alignment with the anterior part,
and reference means (EF) determining the position of said slide (5) and/or of said guide (30) relative to the plate (2) of the tensioning element (1) and therefore determine the interarticular space available in flexion,
said device thus making it possible either to determine the position of the distal femoral cutting plane by determining the difference between the spacing in extension and the space in flexion or, in the case where a distal cut is carried out straightaway, to determine the position of the posterior femoral cutting plane in order to make the spacing in extension and the space in flexion substantially equal.

2. Device according to claim 1, **characterised in that** it comprises a size estimating element (40) which is capable of being mounted on said sliding means (3) so that the size of the femur can be estimated using a reference means and the correct drilling guide element (30) can be chosen.

3. Device according to claim 2, **characterised in that** the size of the plate (2) is such that the femoral end can be grasped between said plate and said element (40), in the manner of a calliper.

4. Device according to any one of claims 1 to 3, **characterised in that** it comprises a distal cutting guide support (50) which has a body (51) capable of sliding on the sliding means (3), from which there extends an arm (54) which will extend parallel to the axis of the knee in flexion, with means for receiving the distal cutting guide and fixing it in a precise location, said precise location being capable of being determined by calculating the difference between the space in flexion and the spacing in extension.

5. Device according to any one of claims 1 to 4, **characterised in that** the tensioning element (1) comprises, extending from said plate (2), sliding means (3) on or in which said slide (5) is capable of sliding, the slide (5) being displaceable by a screw (8) and nut (10) assembly in order to allow the surgeon to make the slide slide and place the knee under tension.

6. Device according to claim 5, **characterised in that** said sliding means (3) have an inner rail in which the slide (5) is guided and an outside surface permitting guiding of said drilling guide (30) and of other elements of the device, said slide having a portion allowing the drilling guide to be moved on the device.

7. Device according to any one of claims 1 to 6, **characterised in that** the drilling guide (30) is arranged to receive a palpating arm which is capable of being brought to bear on the anterior surface of the femoral end in order to limit the depth of penetration of said guide on the guide means, in order to optimise the drilling position.

8. Device according to any one of claims 1 to 7, **characterised in that** the slide (15) has relieved areas (11) permitting precise positioning, relative to the slide, of the plate (21) of the ancillary element (20), and a rapid fixing means (6) which allows said plate (21) to be connected temporarily to the slide (5).

9. Device according to any one of claims 1 to 8, **characterised in that** said size estimating element (40) for palpating the anterior end of the femur is formed in one piece with a body (41) capable of sliding on or in the sliding means (3), the sliding body having a transverse palpating arm (43) which is articulated about an axis (44) parallel to the sliding axis.

## Patentansprüche

1. Vorrichtung zur Anordnung einer Knietotalprothese mit:
einem Teil zum unter Spannung Setzen (1), welches aufweist:
- eine Platte (2), welche sich auf eine Tibialschnittfläche auflegen kann,
- einen Schlitten (5), der auf einer Rutschvorrichtung (3) in eine praktisch senkrecht zu der Platte (2) liegende Richtung bewegt werden kann, und der Vorrichtungen (6, 11) zur vorübergehenden einstückigen Ausbildung mit einem Chirurgiezubehörteil (20) aufweist, das eine zentromedulläre Stange und eine Femoralplatte aufweist, und das Vorrichtungen zur variablen Dickeeinstellung aufnehmen kann, das zuvor am Ende des Oberschenkelknochens angeordnet worden ist, wenn sich das Knie in einer Biegung von ungefähr 90° befindet, und das ermöglicht, einen Abstand bei der Gelenkstreckung zu erhalten,
- und eine Antriebsvorrichtung (8, 10), welche dem Chirurgen ermöglicht, den Schlitten (5) zu bewegen und das Knie unter Spannung zu setzen, wenn die Platte auf den Tibialschnitt aufgelegt und das Chirurgiezubehörteil (20) mit dem Schlitten einstückig ausgebildet ist,
- eine Bohrleitsonde (30), welche auf der Rutschvorrichtung (3) angebracht werden kann und aufweist:
- Bohrlöcher (37), welche die nachträgliche Anordnung eines Schnittblocks auf dem Oberschenkelknochen ermöglichen, wobei der Schnittblock erlaubt, die nachträglichen Femoralschnitte sicherzustellen,
- wobei die Sonde (30) eine Palpationsvorrichtung für den vorderen Bereich des Oberschenkelknochens aufweisen oder damit verbunden sein kann, um die Sonde in Ausrichtung mit diesem vorderen Bereich zu positionieren,
- und Peilvorrichtungen (EF), welche die Position des Schlittens (5) und/oder der Sonde (30) in Bezug zu der Platte (2) des Teils zum unter Spannung Setzen (1) bestimmen und somit den bei der Biegung verfügbaren interartikulären Raum festlegen,
wobei die Vorrichtung somit ermöglicht, entweder die Position der entfernten femoralen Schnittebene durch Bestimmung der Differenz zwischen dem Abstand bei der Gelenkstreckung und dem Raum bei der Biegung zu bestimmen oder im Falle des sofort durchgeführten entfernten Schnittes die Position der nachträglichen Femoralschnittebene zu bestimmen, um eine praktische Gleichheit zwischen dem Streckungsabstand und dem Biegungsraum zu erhalten.

2. Vorrichtung gemäß Anspruch 1, **dadurch gekennzeichnet, dass** sie ein Teil zur Größenabschätzung (40) aufweist, welches auf der Rutschvorrichtung (3) angebracht werden kann, um dank einer Peilvorrichtung die Größe des Oberschenkelknochens schätzen zu können und eine gute Wahl des Bohrsondenteils (30) zu ermöglichen.

3. Vorrichtung gemäß Anspruch 2, **dadurch gekennzeichnet, dass** die Abmessung der Platte (2) derart gestaltet ist, dass das Femoralende zwischen die Platte und das besagte Teil (40) nach Art eines Meßschiebers genommen werden kann.

4. Vorrichtung gemäß einem der Ansprüche 1 bis 3, **dadurch gekennzeichnet, dass** sie einen Träger für die Leitsonde des Fernschnittes (50) aufweist, welcher einen Körper (51) aufweist, der auf der Rutschvorrichtung (3) gleiten kann, und von dem aus sich ein Arm (54) erstreckt, der sich parallel zu der Achse des gebogenen Knies erstreckt, mit Vorrichtungen, um die Leitsonde des Fernschnittes aufzunehmen und an einer bestimmten Stelle zu fixieren, wobei diese bestimmte Stelle durch die Berechnung der Differenz zwischen dem Raum bei der Biegung und dem Abstand bei der Streckung bestimmt werden kann.

5. Vorrichtung gemäß einem der Ansprüche 1 bis 4, **dadurch gekennzeichnet, dass** das Teil zum unter Spannung Setzen (1) Rutschvorrichtungen (3) aufweist, die sich von der besagten Platte (2) aus erstrecken, und auf oder in denen der Schlitten (5) gleiten kann, wobei dieser Schlitten (5) durch eine Schrauben (8)-Mutter (10)-Anordnung beweglich ist, um dem Chirurgen zu ermöglichen, den Schlitten gleiten zu lassen und das Knie unter Spannung zu setzen.

6. Vorrichtung gemäß Anspruch 5, **dadurch gekennzeichnet, dass** die Rutschvorrichtungen (3) eine innere Rutsche aufweisen, in welcher der Schlitten (5) geführt ist, und eine Außenfläche, welche die Führung der besagten Bohrleitsonde (30) und weiterer Teile der Vorrichtung ermöglicht, wobei der Schlitten einen Bereich aufweist, der ermöglicht, die Bohrleitsonde auf der Vorrichtung anzutreiben.

7. Vorrichtung gemäß einem der Ansprüche 1 bis 6, **dadurch gekennzeichnet, dass** die Bohrleitsonde (30) dafür eingerichtet ist, einen Abtastarm aufzunehmen, der sich auf die Vorderfläche des Femoralendes auflegen kann, um die Eindringtiefe der Leitsonde an der Führungsvorrichtung zu begrenzen, um die Bohrposition zu optimieren.

8. Vorrichtung gemäß einem der Ansprüche 1 bis 7, **dadurch gekennzeichnet, dass** der Schlitten (15) Erhöhungen (11) aufweist, welche eine genaue Positionierung der Platte (21) des Chirurgiezubehörteils (20) gegenüber dem Schlitten ermöglichen, und eine Einrichtung zur schnellen Befestigung (6), welche ermöglicht, diese Platte (21) vorübergehend mit dem Schlitten (5) einstückig auszubilden.

9. Vorrichtung gemäß einem der Ansprüche 1 bis 8, **dadurch gekennzeichnet, dass** das Teil zur Größenabschätzung (40) zum Abtasten des vorderen Endes des Oberschenkelknochens von einem Teil mit einem Körper (41) gebildet ist, der auf oder in der Rutschvorrichtung (3) gleiten kann, wobei dieser Gleitkörper einen querliegenden Abtastarm (43) aufweist, der um eine Achse (44) herum gelenkig angebracht ist, welche parallel zu der Gleitachse liegt.
